# EUROPÄISCHE PATENTSCHRIFT

(11) **EP 0 425 938 B1**
(45) Veröffentlichungstag und Bekanntmachung des Hinweises auf die Patenterteilung: **04.05.1994**
(21) Anmeldenummer: 90120095.6
(22) Anmeldetag: 19.10.1990
(51) Int. Cl.: A61F 13/00, A61F 13/10

(54) **Bandagensystem**
Bandage system
Système de bandages

(30) Priorität: 31.10.1989 DE 3936232
(43) Veröffentlichungstag der Anmeldung: 08.05.1991
(73) Patentinhaber: Habermeyer, Peter, Dr., D-70184 Stuttgart (DE)
(72) Erfinder: Habermeyer, Peter, Dr., D-70184 Stuttgart (DE)
(74) Vertreter: Richter, Werdermann & Gerbaulet

(56) Entgegenhaltungen:
- EP-A- 0 198 482
- DE-C- 3 704 288
- US-A- 3 338 236
- US-A- 4 598 703

## Beschreibung

Die Erfindung betrifft eine Kreuzgurtanordnung zur Fixierung der Soll-Relativlage von Schlüsselbein und Schulterblatt im Bereich des Schultereckgelenks mit zwei Gurtbändern.

Das Schultereckgelenk bzw. Acromioclavicula-Gelenk ist eine gelenkige Verbindung zwischen Schlüsselbein und Schulterblatt.

Durch direktes Trauma, z.B. durch einen Sturz vom Fahrrad, kommt es häufig zu Zerreißungen der Gelenkbänder und als Folge davon zu einem schmerzhaften Höherstehen des lateralen Clavicula-Endes. Der Großteil derartiger Verletzungen könnte konservativ behandelt werden, wenn es möglich wäre, das Schlüsselbein mittels einer Haltevorrichtung nach unten zu drücken und in seine gelenkige Verbindung zu reponieren und in dieser Position dauerhaft zu halten.

Durch die US-A-3,338,236 ist eine Kreuzgurtanordnung bekannt, bei der zwei Gurtbänder rückenseitig sich kreuzen, über die Schultern und unterhalb der Achseln, also unterhalb der Schultergelenke in den Spalträumen zwischen diesen und den seitlichen Brustwänden geführt sind, wodurch jedoch die Clavicula nicht ausreichend heruntergedrückt wird. Aufgrund der Gurtbandführung über die Schultergelenke und unterhalb der Achseln wird jedoch ein nach unten gerichteter Zug erreicht. Außerdem sind beide Schultergelenke von den beiden Gurtbändern gleichzeitig beaufschlagt, auch wenn nur ein Schultergelenk einer Fixierung bedarf, so daß kein Tragekomfort gegeben ist, da die unter den Achseln hindurchgeführten Gurtbänder zu Spannungen und Druckbelastungen im Achselbereich führen. Wesentlich ist jedoch, daß bei dieser Kreuzgurtanordnung keine Ruhigstellung des Unterarms erfolgt, so daß das Gewicht des Unterarms zur Erzeugung eines im Bereich des Schultergelenkess erforderlichen Niederhaltedrucks nicht zur Verfügung steht. Auch liegt die Kreuzungsstelle der Gurtbänder nicht im Schlüsselbeinbereich, sondern im Rückenbereich des Körpers des Patienten, so daß die erforderliche Soll-Relativlage vom Schulterblatt und Schlüsselbein im Bereich des Schultergelenkes nicht erreicht werden kann.

Aufgabe der Erfindung ist es, eine Kreuzgurtanordnung zu schafen, die bei einfacher Ausgestaltung, problemfreier Anlegbarkeit und hohem Tragekomfort ein Höhertreten der Clavicula bei gleichzeitigem Vermeiden eines nach unten gerichteten Zugs am Schulterblatt verhindert und somit die geforderte Soll-Relativlage von Schulterblatt und Schlüsselbein im Bereich des Schultereckgelenks sicherstellt.

Diese Aufgabe wird durch die im Anspruch 1 gekennzeichneten Merkmale gelöst.

Bei einer derart ausgebildeten Kreuzgurtanordnung ist die Kreuzungsstelle der Gurtbänder im Bereich des Schlüsselbeins gelegen, wobei die wechselseitig einander zugeordneten freien Enden der Gurtbänder paarweise einerseits im Bereich des distalen Oberarms und andererseits im Bereich des Handteils des abgewinkelt vor dem Oberkörper fixierten Unterarms befestigt sind.

Durch die Ausnutzung des bezüglich des Oberkörpers im wesentlichen ruhig gestellten Unterarms zur Fixierung des Bandagensystems und die dabei gleichzeitig erreichte Ausnutzung des Gewichts des Unterarms zur Erzeugung des im Bereich des Schultereckgelenks erforderlichen Niederhaltedrucks wird eine sehr wirksame, einen sicheren Sitz aufweisende und auch einen hohen Tragekomfort gewährleistende Acromioclavicula-Gelenkbandage erhalten.

Eine besonders vorteilhafte Ausführungsform der Erfindung zeichnet sich dadurch aus, daß die Kreuzgurtanordnung aus einem ersten Gurtband und einem zweiten Gurtband besteht, daß das erste Gurtband ausgehend von einer Befestigungsstelle am vorderseitigen Bereich des distalen Oberarms über das Schlüsselbein, den Nacken und quer über den Rücken zu einer Befestigungsstelle im Bereich des Handteils verläuft, und daß sich das zweite Gurtband ausgehend von einer Befestigungsstelle am rückseitigen Bereich des distalen Oberarms über die Schulter und das Schlüsselbein zu einer ebenfalls im Bereich des Handteils vorgesehenen Befestigungsstelle erstreckt.

Hinsichtlich der einfachen Anlegbarkeit der Kreuzgurtanordnung nach der Erfindung und auch hinsichtlich der exakten Festlegung der Befestigungsstellen für die Gurtbänder ist es von besonderem Vorteil, die Befestigungsstellen an einer Unterarmbandage in Form eines sich vom Ellbogenbereich bis zum Handbereich erstreckenden Schlauchteils vorzusehen.

Die Unterarmbandage weist vorzugsweise an ihrem von der Handseite abgewandten Ende einen sich um den unteren Bereich des Oberarms erstreckenden Befestigungsbund für die beiden Gurtbänder auf, und dieser Befestigungsbund ist insbesondere verstärkt ausgebildet und kann über einen Klettverschluß schließbar sein.

Die Kreuzgurtanordnung ist sowohl für die linke als auch für die rechte Seite verwendbar.

Weitere besonders vorteilhafte Merkmale der Erfindung sind in den Unteransprüchen angegeben.

Ein Ausführungsbeispiel der Erfindung wird unter Bezugnahme auf die Zeichnung näher erläutert. Es zeigt
Fig. 1 eine Vorderansicht der Kreuzgurtanordnung im angelegten Zustand,
Fig. 2 eine Seitenansicht der Kreuzgurtanordnung nach Fig. 1,
Fig. 3 eine Rückansicht der Kreuzgurtanordnung nach Fig. 1, und
Fig. 4 eine schmematische Darstellung der Kreuzgurtanordnung im nicht angelegten Zustand.

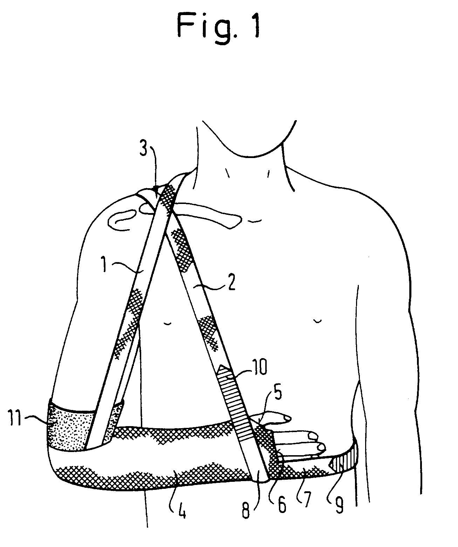

Fig. 1 zeigt, daß durch die Kreuzgurtanordnung der Unterarm der betroffenen Extremität am Oberkörper ruhiggestellt wird. Dazu weist die Kreuzgurtanordnung als Bandagensystem zwei Gurtbänder 1,2 auf, die sich über dem Schlüsselbein im Bereich des Schultereckgelenks an der mit dem Bezugszeichen 3 gekennzeichneten Stelle kreuzen und sicherstellen, daß einerseits kein Zug am Schulterblatt nach unten und andererseits kein Höhertreten der Clavicula entstehen kann.

Der erste Gurt 1 ist im Bereich des distalen Oberarms vorderseitig an einem Befestigungsbund 11 angebracht und läuft über das Schlüsselbein, den Naken und quer über den Rücken in den Bereich des Handteiles, wo er mit einem Anschlußteil 7 über einen Klettverschluß 9 verbunden ist, das Bestandteil einer schlauchförmigen Unterarmbandage 4 ist, an der auch der Befestigungsbund 11 angebracht ist. Der Befestigungsbund 11 und die Unterarmbandage 4 können natürlich einteilig ausgeführt sein, wobei der Befestigungsbund 11 stets so zu gestalten ist, daß die erforderliche Festigkeit gegeben ist. Wenn aufgrund der erforderlichen Festigkeit die Radialelastizität des Befestigungsbundes 11 nicht mehr ausreichend ist, dann wird dieser Befestigungsbund 11 als ein vorzugsweise über einen Klettverschluß verschließbares Teil ausgebildet, das aber weiterhin mit der Unterarmbandage 4 fest verbunden ist, um sicherzustellen, daß die Zugkräfte aufgenommen werden können, ohne daß ein störend festes Anliegen des Befestigungsbundes 11 am Oberarm gefordert werden muß.

Das zweite Gurtband 2 ist am rückwärtigen Teil des Befestigungsbundes 11 im Bereich des distalen Oberarms angebracht, kreuzt von hinten nach vorne über die Schulter und wird am Unterarm im Handbereich befestigt. Die Befestigung erfolgt dabei vorzugsweise über eine Schlaufe 8, die aufgrund einer vorgesehenen Klettverschlußverbindung 10 jeweils passend einstellbar ist. Die Schlaufe 8 kann in ihrer Position beispielsweise durch eine an der Unterarmbandage 4 vorgesehene Führungsschlinge oder auch durch einen Klettverschlußbereich zwischen Schlinge und Unterarmbandage festgelegt werden, wenn dies für erforderlich gehalten wird.

Das handseitige Ende der schlauchförmigen Unterarmbandage 4 weist bevorzugt eine Daumendurchtrittsöffnung 5 und eine Fingerdurchtrittsöffnung 6 auf, wobei das Anschlußteil 7 zur Verbindung mit dem ersten Gurtband 1 im unteren Bereich, d.h. etwa im Bereich des kleinen Fingers, gelegen ist.

Aufgrund der Klettverschlußverbindungen 9,10 läßt sich die Gelenkbandage den jeweiligen Gegebenheiten problemfrei anpassen, und durch das Vorsehen aller Befestigungsstellen der Gurtbänder an der schlauchförmigen Unterarmbandage 4 werden stets definierte Anschlußstellen für die Gurte gewährleistet, die wiederum sicherstellen, daß im Bereich der Gurtkreuzungsstelle 3 die geforderten Niederhaltekräfte für das Schlüsselbein wirksam werden, ohne daß gleichzeitig ein Zug am Schulterblatt nach unten auftritt.

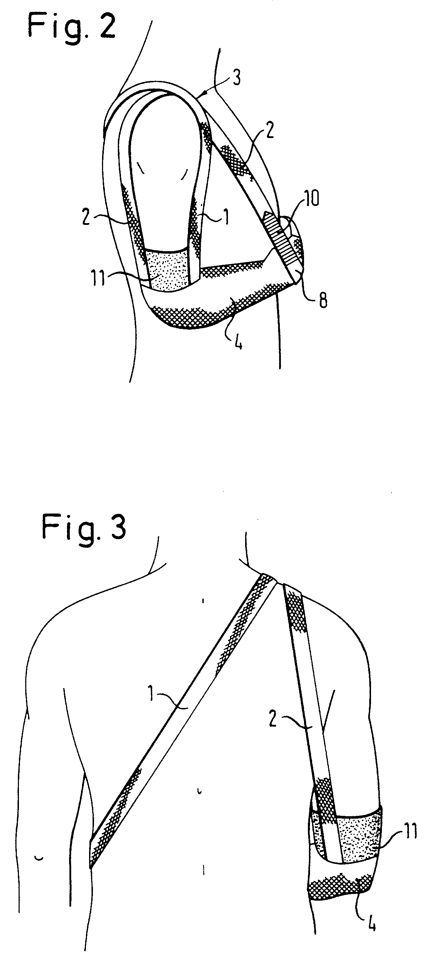

Fig. 2 zeigt die Kreuzgurtanordnung nach Fig.1 in einer Seitenansicht und läßt besonders gut den Verlauf des Gurtbandes 2 von der Rückseite des Befestigungsbundes 11 zum Handbereich der Unterarmbandage 4 erkennen.

Fig. 3 zeigt den Verlauf der Gurtbänder 1 und 2 am Rücken des jeweiligen Patienten. Die Fig. 1 und 3 machen dabei besonders deutlich, daß die Gurte 1 und 2 so verlaufen, daß praktisch keinerlei Tendenzen zum Verrutschen gegeben sein können und somit der für die Druckausübung wichtige Kreuzungsbereich 3 in seiner Lage eindeutig definiert ist.

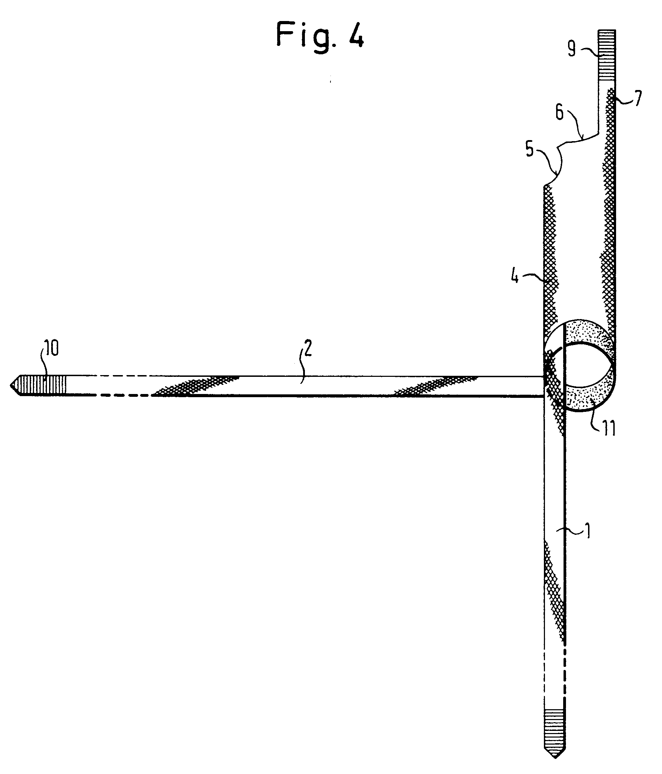

Die schematische Darstellung nach Fig.4 zeigt den äußerst einfachen und damit auch eine kostengünstige Fertigung ermöglichenden Grundaufbau der Kreuzgurtanordnung.

Die Kreuzgurtanordnung umfaßt - wie im angelegten Zustand bereits erläutert - eine schlauchförmige, insbesondere radialelastisch ausgebildete Unterarmbandage 4, die an einem Ende vorzugsweise verstärkt ausgebildet ist, um einen Befestigungsbund 11 zu schaffen, an dem die beiden Gurtbänder 1,2 angenäht werden können. Der Verlauf des Gurtbandes 2 ist nur zur Verdeutlichung der Zeichnung abgewinkelt dargestellt.

Beide Enden der Gurtbänder 1 und 2 sind mit Klettverschlußelementen versehen, wobei mit dem Ende des Gurtbandes 2 die in Fig. 1 gezeigte Schlaufe 8 gebildet wird, während das Ende des Gurtbandes 1 mit dem Klettverschluß 9 an dem Anschlußteil 7 verbunden werden kann, das an dem dem Befestigungsbund 11 gegenüberliegenden Ende der Unterarmbandage 4 vorgesehen ist.

Um eine eindeutige Lage des Anschlußteils 7 im angelegten Zustand der Bandage vorzugeben, sind am handseitigen Ende der Unterarmbandage 4 eine Daumendurchtrittsöffnung 5 und eine Fingerdurchtrittsöffnung 6 vorgesehen.

Die Kreuzgurtanordnung läßt in der gezeigten Ausgestaltung im angelegten Zustand eine gewisse Beweglichkeit des Ellbogengelenks vom Körper weg und zum Körper hin zu. Wenn diese Beweglichkeit nicht gewünscht wird, kann ein dritter Gurt vorgesehen werden, der sich dann von dem Befestigungsbund 11 quer über den Rücken zum Handbereich erstreckt und dort analog dem Gurt 1 befestigt werden kann.

## Patentansprüche

1. Kreuzgurtanordnung zur Fixierung der Soll-Relativlage von Schlüsselbein und Schulterblatt im Bereich des Schultereckgelenks mit zwei Gurtbändern (1,2), dadurch gekennzeichnet, daß sie eine Unterarmbandage (4) mit einem an ihrem der Hand abgekehrten Ende vorgesehenen Befestigungsbund (11) aufweist, an dem die beiden Gurtbänder (1,2) des Kreuzgurtes angeordnet sind und deren wechselseitig einander zugeordnete freie Enden paarweise einerseits im Bereich des distalen Oberarms und andererseits im Bereich des Handteils des abgewinkelt vor dem Oberkörper fixierten Unterarms befestigbar angeordnet sind, so daß die Kreuzungsstelle (3) im Bereich des Schlüsselbeins gelegen ist.

2. Kreuzgurtanordnung nach Anspruch 1, dadurch gekennzeichnet, daß die Kreuzgurtanordnung aus einem ersten Gurtband (1) und einem zweiten Gurtband (2) besteht, daß das erste Gurtband (1) ausgehend von einer Befestigungsstelle am vorderseitigen Bereich des distalen Oberarms über das Schlüsselbein, den Naken und quer über den Rücken zu einer Befestigungsstelle im Bereich des Handteils verläuft, und daß sich das zweite Gurtband (2) ausgehend von einer Befestigungsstelle am rückwärtigen Bereich des distalen Oberarms über die Schulter und das Schlüsselbein zu einer ebenfalls im Bereich des Handteils vorgesehenen Befestigungsstelle erstreckt.

3. Kreuzgurtanordnung nach Anspruch 1 oder 2, dadurch gekennzeichnet, daß die Befestigungsstellen beider Gurtbänder (1,2) an einer Unterarmbandage (4) vorgesehen sind, wobei insbesondere die Unterarmbandage (4) aus einem insbesondere radial-elastisch ausgebildeten Schlauchteil besteht.

4. Kreuzgurtanordnung nach einem der Ansprüche 1 bis 3, dadurch gekennzeichnet, daß die schlauchförmige Unterarmbandage (4) im Handbereich zumindest eine Durchtrittsöffnung für Finger und Daumen sowie ein Anschlußteil (7) zur lösbaren Verbindung mit dem ersten Gurtband (1) aufweist, wobei insbesondere die Durchtrittsöffnung für Finger und Daumen in eine Fingerdurchtrittsöffnung (6) und eine Daumendurchtrittsöffnung (5) unterteilt ist.

5. Kreuzgurtanordnung nach einem der Ansprüche 1 bis 4, dadurch gekennzeichnet, daß in dem die Hand aufnehmenden Bereich der schlauchförmigen Unterarmbandage (4) zumindest ein Halteelement zur lösbaren Befestigung des zweiten Gurtbandes (2) vorgesehen ist.

6. Kreuzgurtanordnung nach einem der Ansprüche 1 bis 4, dadurch gekennzeichnet, daß das zur handseitigen Befestigung bestimmte Ende des zweiten Gurtbandes (2) als verstellbare Schlaufe (8) ausgebildet ist.

7. Kreuzgurtanordnung nach einem der Ansprüche 1 bis 6 , dadurch gekennzeichnet, daß das Anschlußteil (7) und das erste Gurtband (1) über einen Klettverschluß (9) verbindbar sind.

8. Kreuzgurtanordnung nach einem der Ansprüche 1 bis 7 , dadurch gekennzeichnet, daß das freie Ende des zweiten Gurtbandes (2) mittels eines Klettverschlusses (10) in Schlaufenform im Handbereich fixierbar ist, wobei vorzugsweise die Relativlage zwischen der mittels des freien Endes des zweiten Gurtbandes (2) gebildeten Schlaufe (8) und der Unterarmbandage (4) mittels zumindest einer Schlinge oder durch einen zwischen diesen beiden Teilen vorgesehenen Klettverschlußbereich bestimmt ist.

9. Kreuzgurtanordnung nach einem der Ansprüche 1 bis 8 , dadurch gekennzeichnet, daß im Bereich der Kreuzungsstelle (3) zwischen den beiden Gurtbändern (1,2) Klettverschlußbereiche zur gegenseitigen Fixierung der beiden Gurtbänder (1,2) vorgesehen sind.

10. Kreuzgurtanordnung nach einem der Ansprüche 1 bis 9, dadurch gekennzeichnet, daß die Unterarmbandage (4) an ihrem von der Handseite abgewandten Ende einen sich um den unteren Bereich des Oberarms erstreckenden Befestigungsbund (11) für die beiden Gurtbänder (1,2) aufweist, wobei insbesondere der Befestigungsbund (11) verstärkt ausgebildet und insbesondere über einen Klettverschluß schließbar ist, und/oder daß ein drittes Gurtband zur Fixierung des Ellbogenbereichs vorgesehen ist, das sich vom Befestigungsbund (11) quer über den Rücken zum Handbereich erstreckt.

## Claims

1. Bandage system for the fixation of the correct relative position of clavicle and scapula within the region of the acromioclavicular joint with two strap bands (1,2),
characterized in that
it possesses an antebrachium bandage (4) with an attachment band (11) on its end facing away from the hand, on which the two strap bands (1,2) of the cross strap are disposed and whose free ends which are alternately allocated to each other are disposed in pairs, on the one hand, in the region of the distal brachium and, on the other hand, in the region of the hand portion of the antebrachium, is detachably fixed so as to be angled in front of the trunk, so that the crossing point (3) is located in the region of the clavicle.

2. Bandage system according to Claim 1,
characterized in that
the cross strap is comprised of a first strap band (1) and a second strap band (2), in that the first strap band (1), starting from an attachment point on the front-side area of the distal brachium, proceeds via the clavicle, the nucha and transversely across the dorsum to an attachment point in the region of the hand portion, and in that the second strap band (2), starting out from an attachment point on the rearward region of the distal brachium, extends across the shoulder and the clavicle to an attachment point likewise provided in the area of the hand portion.

3. Bandage system according to either Claim 1 or 2,
characterized in that
the attachment points of both strap bands (1,2) are provided on an ante brachium bandage (4), in which case particularly the antebrachium bandage (4) is comprised of an especially constructed radial-elastc tubular portion.

4. Bandage system according to any of Claims 1 to 3,
characterized in that
the tubular antebrachium bandage (4), in the hand region, possesses at least one opening for the fingers and thumb as well as a connecting portion (7) for a detachable connection with the first strap band (1), in which case particularly the opening for fingers and thumb is subdivided into a finger aperture (6) and a thumb aperture (5).

5. Bandage system according to any of Claims 1 to 4,
characterized in that,
in the region of the tubular antebrachium bandage (4) accommodating the hand, at least one retaining element for the detachable attachment of the second strap band (2) is provided.

6. Bandage system according to any of Claims 1 to 4,
characterized in that
the end of the second strap band (2) intended for the attachment on the hand side is constructed in the form of an adjustable loop (8).

7. Bandage system according to any of Claims 1 to 6,
characterized in that
the connecting portion (7) and the first strap band (1) can be connected with the aid of a Velcro strip fastener.

8. Bandage system according to any of Claims 1 to 7,
characterized in that
the free end of the second strap band (2) can be fixed with the aid of a Velcro strip fastener (10) in the form of a loop in the hand region, in which case, by preference, the relative position between the loop (8) formed by means of the free end of the second strap band (2) and the antebrachium bandage (4) is determined with the aid of at least one loop or by means of a Velcro strip fastener area provided between these two parts.

9. Bandage system according to any of Claims 1 to 8,
characterized in that,
in the area of the crossing point (3) between the two strap bands (1,2), Velcro strip fastener areas are provided for the reciprocal fixation of the two strap bands (1,2).

10. Bandage system according to any of Claims 1 to 9,
characterized in that
the antebrachium bandage (4), on its end facing away from the hand side, possesses an attachment band (11) extending around the lower region of the brachium for the two strap bands (1,2), in which case particularly the attachment band (11) is constructed in a reinforced fashion and, more particularly, can be closed with the aid of a Velcro strip fastener, and/or in that a third strap band for the fixation of the cubitus region is provided, which extends from the attachment band (11) transversely across the dorsum to the hand region.

## Revendications

1. Dispositif de sangles croisées pour la fixation de la position relative nominale de la clavicule et de l'omoplate dans la zone de l'articulation de jointure de l'épaule avec deux sangles (1, 2), **caractérisé en ce** qu'il présente un bandage de l'avant-bras (4) avec un bandeau de fixation (11), prévu à son extrémité détournée de la main, sur lequel les deux sangles (1, 2) des sangles croisées sont placées et dont les extrémités libres qui correspondent l'une à l'autre en alternance sont disposées en pouvant être fixées par paires d'une part dans la zone du bras distal et d'autre part dans la zone de la partie main de l'avant-bras fixé en étant coudé devant le haut du corps si bien que l'endroit de croisement (3) est situé dans la zone de la clavicule.

2. Dispositif de sangles croisées selon la revendication 1, **caractérisé en ce** que le dispositif de sangles croisées est constitué par une première sangle (1) et une seconde sangle (2), que la première sangle (1) part d'un endroit de fixation dans la zone avant du bras distal, en passant par la clavicule, la nuque, en traversant le dos, vers un endroit de fixation dans la zone de la partie main et que la seconde sangle (2) part d'un endroit de fixation dans la zone arrière du bras distal, passe par l'épaule et la clavicule, pour s'étendre vers un endroit de fixation également prévu dans la zone de la partie main.

3. Dispositif de sangles croisées selon la revendication 1 ou 2, **caractérisé en ce** que les endroits de fixation des deux sangles (1, 2) sont prévus sur un bandage de l'avant-bras (4), le bandage de l'avant-bras (4) étant en particulier constitué par une partie gaine en particulier configurée en étant élastique dans le sens radial.

4. Dispositif de sangles croisées selon l'une des revendications 1 à 3, **caractérisé en ce** que le bandage de l'avant-bras (4) en forme de gaine présente dans la zone de la main au moins une ouverture de passage pour les doigts et le pouce ainsi qu'une partie raccord (7) pour la jonction amovible avec la première sangle (1), l'ouverture de passage pour les doigts et le pouce étant divisée en une ouverture de passage pour les doigts (6) et une ouverture de passage pour le pouce (5).

5. Dispositif de sangles croisées selon l'une des revendications 1 à 4, **caractérisé en ce** qu'au moins un élément de retenue est prévu dans la zone qui loge la main du bandage de l'avant-bras en forme de gaine (4) pour la fixation amovible de la seconde sangle (2).

6. Dispositif de sangles croisées selon l'une des revendications 1 à 4**, caractérisé en ce** que l'extrémité de la seconde sangle (2), qui est destinée à la fixation côté main, est configurée comme une boucle réglable (8).

7. Dispositif de sangles croisées selon l'une des revendications 1 à 6, **caractérisé en ce** que la partie raccord (7) et la première sangle (1) peuvent être reliées par une fermeture velcro (9).

8. Dispositif de sangles croisées selon l'une des revendications 1 à 7, **caractérisé en ce** que l'extrémité libre de la seconde sangle (2) peut être fixée au moyen d'une fermeture velcro (10) en forme de boucle dans la zone de la main, la position relative entre la boucle (8) formée au moyen de l'extrémité libre de la seconde sangle (2) et le bandage de l'avant-bras (4) étant de préférence déterminée au moyen d'au moins une boucle ou par une zone de fermeture velcro prévue entre ces deux parties.

9. Dispositif de sangles croisées selon l'une des revendications 1 à 8, **caractérisé en ce** que des zones de fermeture velcro sont prévues dans la zone de l'endroit de croisement (3) entre les deux sangles (1, 2) pour la fixation mutuelle des deux sangles (1, 2).

10. Dispositif de sangles croisées selon l'une des revendications 1 à 9, **caractérisé en ce** que le bandage de l'avant-bras (4) présente, à son extrémité détournée du côté main, un bandeau de fixation (11) pour les deux sangles (1, 2) qui s'étend autour de la zone inférieure du bras, le bandeau de fixation (11) étant en particulier configuré en étant renforcé et en pouvant être fermé en particulier par une fermeture velcro et/ou qu'il est prévu une troisième sangle pour la fixation de la zone du coude qui s'étend du bandeau de fixation (11) vers la zone de la main en traversant le dos.
